# EUROPEAN PATENT SPECIFICATION

(11) **EP 2 010 676 B1**
(45) Date of publication and mention of the grant of the patent: **09.01.2013**
(21) Application number: 07748437.6
(22) Date of filing: 26.04.2007
(51) Int. Cl.: C12Q 1/68

(54) **METHOD AND KIT FOR MOLECULAR CHROMOSOMAL QUANTIFICATION**
VERFAHREN UND KIT ZUR MOLEKULAREN CHROMOSOMENQUANTIFIZIERUNG
PROCEDE ET KIT POUR QUANTIFICATION MOLECULAIRE DE CHROMOSOMES

(30) Priority: 27.04.2006 US 795159 P
(43) Date of publication of application: 07.01.2009
(73) Proprietor: Vytal Diagnostics AB, 106 91 Stockholm (SE)
(72) Inventor: HAUZENBERGER, Dan, 132 35 Saltsjö-Boo (SE); KLANGBY, Ulf, 192 76 Sollentuna (SE); HEDRUM, Anders, 138 35 Älta (SE)
(74) Representative: Berg, Anna Margareta
(86) International application number: PCT/SE2007/050276
(87) International publication number: WO 2007/126377

(56) References cited:
- WO-A2-02/068685
- US-A1- 2003 054 386
- HULTEN M A ET AL.: "Rapid and simple prenatal diagnosis of common chromosome disorders: advantages and disadvantages of the molecular methods FISH and QF-PCR" REPRODUCTION, vol. 126, 2003, pages 279-297, XP002559856
- CIRIGLIANO V ET AL.: "RAPID PRENATAL DIAGNOSIS OF COMMON CHROMSOME ANEUPLOIDIES BY QF-PCR. ASSESSMENT ON 18000 CONSECUTIVE CLINICAL SAMPLES" MOLECULAR HUMAN REPRODUCTION, vol. 10, 2004, pages 839-846, XP002559857
- White H: "ChromoQuantTM (version 1) in vitro diagnostic test kit for analysis of common chromosomal disorders" National Genetics Reference Laboratory (Wessex) September 2005 (2005-09), XP002559858 Retrieved from the Internet: URL:http://www.ngrl.co.uk/Wessex/downloads /pdf/CQ1.pdf> [retrieved on 2009-12-09]
- CIRIGLIANO V ET AL: "X CHROMOSOME DOSAGE BY QUANTITATIVE FLUORESCENT PCR AND RAPID PRENATAL DIAGNOSIS OF SEX CHROMOSOME ANEUPLOIDIES" MOLECULAR HUMAN REPRODUCTION, OXFORD UNIVERSITY PRESS, GB - BE, vol. 8, no. 11, 1 November 2002 (2002-11-01), pages 1042-1045, XP009019721 ISSN: 1360-9947
- DONAGHUE C ET AL: "DEVELOPMENT AND TARGETED APPLICATION OF A RAPID QF-PCR TEST FOR SEX CHROMOSOME IMBALANCE" PRENATAL DIAGNOSIS, CHICHESTER, SUSSEX, GB, vol. 23, no. 3, 3 February 2003 (2003-02-03), pages 201-210, XP009019715 ISSN: 0197-3851
- NICOLINI U ET AL.: "The introduction of QF-PCR in prenatal diagnosis of fetal aneuploidies: time for reconsideration" HUMAN REPRODUCTION UPDATE, vol. 10, 2004, pages 541-548, XP002559859
- CIRIGLIANO V ET AL.: "Rapid prenatal diagnosis by QF-PCR: Evaluation of 30,000 consecutive clinical samples and future applications" ANNALS OF THE NEW YORK ACADEMY OF SCIENCES, vol. 1075, September 2006 (2006-09), pages 288-298, XP002559860
- EYCHENE A. ET AL.: 'Chromosomal assignment of two human B-raf (Rmil) proto-oncogene loci: B-raf-1 encoding the p94(Braf/Rmil) and B-raf-2, a processed pseudogene' ONCOGENE vol. 7, 1992, pages 1657 - 1660, XP003015783
- LEE H.H. ET AL.: 'Rapid detection of trisomy 21 by homologous gene quantitative PCR (HGQ-PCR)' HUM. GENET. vol. 99, 1997, pages 364 - 367, XP003015784
- CIRIGLIANO V. ET AL.: 'Clinical application of multiplex quantitative fluorescent polymerase chain reaction (QF-PCR) for the rapid prenatal detection of common chromosome aneuploidies' MOLECULAR HUMAN REPRODUCTION vol. 7, no. 10, 2001, pages 1001 - 1006, XP003015785

## Description

### Field of the invention

The present invention relates to a novel method for use in the detection or diagnosis of chromosomal abnormalities. The invention in particular relates to a method for relative molecular quantification of chromosomes enabling the detection of both chromosomal aneuploidies as well as quantitative and qualitative gene aberrations. The invention further relates to a diagnostic kit.

### Background

It is well known that the risk of foetal chromosomal anomalies (e.g. Down's syndrome) increases with maternal age. Prenatal diagnostics answers the need to detect early in pregnancy a number of chromosomal anomalies. The prenatal diagnosis of chromosomal anomalies has become widely available for pregnancies at risk in the last three decades. The risk increases with age and a markedly increased risk is seen in mothers aged 35 or more. Chromosomal anomalies frequently involve trisomy 21 (Down's syndrome), but also trisomies 13 and 18 and sex chromosome defects are frequently observed in children born by mothers in this age group. At the age of 20 the risk of trisomy 21 is approximately 1/2000,1/1200 at 25,1/900 at 30,1/400 at 35, 1/100 at 40 and 1/40 at 45 years of age.

Karyotyping is the most frequently used test method on material obtained by invasive-techniques such as CVS and amniocentesis. Karyotyping detects a range of numerical and structural chromosome abnormalities in addition to the common autosomal trisomies 13 (Patau's syndrome), 18 (Edwards' syndrome) and 21 (Down's syndrome) as well as sex chromosome abnormalities e.g. X0 (Turner's syndrome) and XXY (Klinefelter's syndrome). However, since amniotic fluid and chorionic villus cells are cultured before analysis, delays of up to 14 days or longer can occur before the results are available. Molecular methods based on Polymerase Chain Reaction (PCR) and DNA probe hybridisation have therefore been developed ( Non Patent Citation 0001: HULTEN, M A. Rapid and simple prenatal diagnosis of common chromosome disorders: advantages and disadvantages of the molecular methods FISH and QF-PCR. Reproduction, 2003 vol. 126, no. 3, p. 279-97.
and
Non Patent Citation 0002: NICOLINI, U. The introduction of QF-PCR in prenatal diagnosis of fetal aneuploidies: time for reconsideration. Human Reproduction Update, 2004 vol. 10, no. 6, p. 541-548. ). These techniques are faster than karyotyping and can be used for the common autosomal and sex chromosome aneuploidies mentioned above. Among the techniques currently used are FISH (fluorescent-in-situ-hybridization) of non-cultured cells and QF-PCR (Quantitative Flourescent PCR). These techniques generate results within 48 hrs but may have limitations in detecting chromosomal aneuploidies within mosaicism
(Non Patent Citation 0003: CAINE, A. Prenatal detection of Down's syndrome by rapid aneuploidy testing for chromosomes 13, 18, and 21 by FISH or PCR without a full karyotype: a cytogenetic risk assessment. Lancet, 2005 vol. 366, no. 9480, p. 123-8.
) and/or in samples with maternal cell contamination (MCC). However, the fact that these test generate results within 24-48 hrs enabling early decisions on pregnancy management for abnormal foetuses has led to a technique shift for prenatal screening in many laboratories.

QF-PCR is based on a technology where chromosome-specific, repeated DNA sequences (known as short tandem repeats (STRs) are amplified by PCR. The use of fluorescently labelled primers allows visualisation and quantification of the fluorescently labeled PCR products. Quantification may be performed by calculating the ratio of the specific peak areas of the respective repeat lengths using an automated DNA sequencer. STRs vary in length between subjects, depending on the number of times the tri-, tetra- or penta-nucleotides are repeated. DNA amplified from normal subjects who are heterozygous (have alleles of different lengths) is expected to show two peaks with the same peak areas. DNA amplified from subjects who are trisomic will exhibit either an extra peak (being triallelic) with the same area, or only two peaks (being diallelic), one of them twice as large as the other (
Non Patent Citation 0004: NICOLINI, U. The introduction of QF-PCR in prenatal diagnosis of fetal aneuploidies: time for reconsideration. Human Reproduction Update, 2004 vol. 10, no. 6, p. 541-548.
). Subjects who are homozygous (have alleles of same length) or monsosomic will display only one peak.

The inability of the QF-PCR technique to distinguish subjects who are homozygous or monosomic is a major shortcoming when testing for sex chromosome abnormalities. When STRs specific for chromosome X are used, some samples from normal females (46,XX) may show homozygous QF-PCR patterns, indistinguishable from those produced by samples with a single X, as in Turner's syndrome (45,X). Incorporating additional X-chromosome STR markers into the analysis will reduce but not eliminate the likelihood of homozygosity (
Non Patent Citation 0005: DONAGHUE, C. Development and targeted application of a rapid QF-PCR test for sex chromosome imbalance. Prenat Diagn, 2003 vol. 23, no. 3, p. 201-10).

Prenatal diagnosis of Turner's syndrome by determining the abscence of a methylated copy of the FMR-1 gene on the X-chromosome has been described as potentially useful method for detection of Turner's syndrome (
Non Patent Citation 0006: PENA, S D J. Fetal diagnosis of monosomy X (Turner's syndrome) with methylation-specific PCR. Prenatal Diagnosis, 2003 vol. 23, p. 769-770).

Genotyping the X-Y homologous amelogenin (AMELX and AMELY) gene segments for gender identification is widely used for DNA profiling in prenatal diagnoses. Regions on this gene are sufficiently conserved and may be amplified, using identical primers, for simultaneous detection of the AMELX and AMELY alleles in gender identification procedures. When amplification of AMELX and AMELY is used in the QF-PCR procedure it may also be helpful in providing a quantitative relationship between chromosomes X and Y. However, no quantitative information for the X-chromosome will be obtained in females as the AMELY gene is not present.

Relative quantification of the X-chromosome by co-amplification of a X-chromosome STR marker (HPRT) versus a chromosome 21 (D21S 1411) STR marker using separate primer pairs has been suggested as an alternative strategy for QF-PCR to diagnose monosomy X. The method does not require a heterozygous pattern to he obtained for the STR markers but assumes an identical amplification efficiency of the two different primer pairs amplifying two different STR markers (
Non Patent Citation 0007: CIRIGLIANO, V. X chromosome dosage by quantitative fluorescent PCR and rapid prenatal diagnosis of sex chromosome aneuploidies. Molecular Human reproduction, 2002 vol. 8, no. 11, p. 1042-1045. ).

Cirigliano et al. have similarly used STR markers and QF-PCR for prenatal diagnosis of chromosome abnormalities (Molecular Human Reproduction, "Rapid prenatal diagnosis of chromosome aneuploidies by QF-PCR. Assessment on 18 000 consecutive clinical samples.", vol. 10, 2004, p. 839-846 and Annals of the New York Academy of Sciences, "Rapid prenatal diagnosis by QF-PCR: Evaluation of 30,000 concecutive clinical samples and future applications", vol. 1075, September 2006, p. 288-298.)

An in vitro diagnostic test kit "ChromoQuant^{™}" for analysis of chromosomal disorders are provided by Cybergene AB and evaluated by National Genetics Reference Laboratory (Wessex), XP-002559858, September 2005. ChromoQuant^{™} relies on the detection of heterozygous STR markers and non-variable sex determining markers. A major shortcoming with this technique is the need to detect heterozygous markers in order to detect the ploidy of chromosomes.

Consequently there remains a need for an improved method setting aside the shortcomings and disadvantages associated with known methods.

### Summary

In developing a method for quantification of chromosomes and genes in a sample taken from human the present inventors have surprisingly found at the above shortcomings and disadvantages can be set aside by choosing at least two non-STR marker sequences, wherein one marker sequence is a sequence known to be present on the chromosome X, another marker sequence is a sequence known to be present on an autosomal chromosome, and the marker sequences are partially homologous. According to this method, the sample is amplified using substantially homologous PCR primer pairs hybridising to the marker sequences known to be present on the chromosomes, and amplified DNA fragments are detected.

A ratio of the amplification products of the marker sequences present on the autosomal chromosome and chromosome X which is 2:1 is indicative of Turners syndrome.

The invention is defined in the attached claims, incorporated herein by reference.

### Short description of the drawings

The invention will be described in closer detail in the following description, nonlimiting examples and claims, with reference to the attached drawings in which

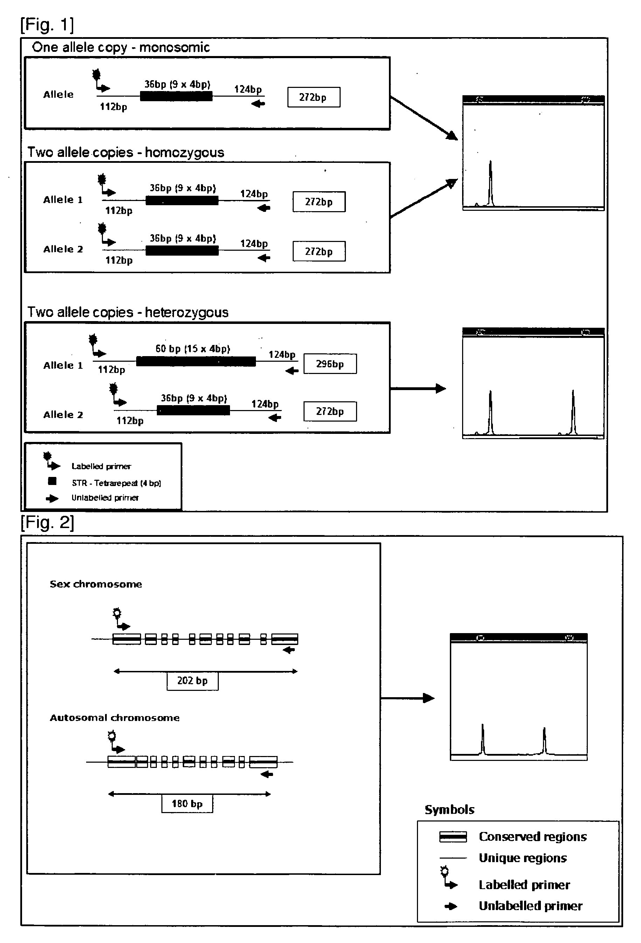

Figure 1 illustrates an analysis according to the background technology where, using QF-PCR and chromosome specific STRs, it is not possible to distinguish between subjects who are homozygous or monosomic. In this test, normal heterozygous subjects will display two peaks with the same peak area. DNA amplified from trisomic subjects will exhibit an extra peak (being triallelic), or only two peaks (being diallelic), whereas subjects who are homozygous or monosomic will display only one peak.

Figure 2 illustrates an embodiment of the invention where one primer pair is used, specific for a marker sequence on a sex chromosome, as well as for a marker sequence on an autosomal chromosome, the two marker sequences being at least partially homologous and of different length.

Figure 3a shows a normal Male (46,XY) chromatogram as displayed by the presence of AMELX and AMELY in a 1:1 ratio. A single allele of the X-specific DXS1187 marker and a 2:1 area ratio of chromosome 7 (BRAF7) to chromosome X (BRAFX).

Figure 3b shows a normal female (46,XX) chromatogram as displayed by the presence of AMELX and absence of AMELY. A two allele pattern in a 1:1 ratio of the X-specific DXS1187 marker and a 1:1 area ratio of chromosome 7 (BRAF7) to chromosome X (BRAFX).

Figure 3c shows a Turner Syndrom, X0 (45,X), female chromatogram as displayed by the presence of AMELX and absence of AMELY. A one allele pattern of the X-specific DXS1187 marker and a 2:1 area ratio of chromosome 7 (BRAF7) to chromosome X (BRAFX).

### Detailed description

It must also be noted that, as used in this specification and the appended claims, the singular forms "a", "an", and "the" include plural referents unless the context clearly dictates otherwise.

The term "about" when used in the context of numeric values denotes an interval of accuracy, familiar and acceptable to a person skilled in the relevant art. Said interval can be ± 10 % or preferably ± 5 %.

In describing and claiming the present invention, the following terminology will be used in accordance with the definitions set out herein.

The term "sample" here means a volume of a liquid, solution, biopsy or cell suspension, taken from a human. The sample may be subjected to qualitative or quantitative determination according to the invention as such, or after suitable pre-treatment, such as homogenisation, sonication, filtering, sedimentation, centrifugation, etc.

Typical samples in the context of the present invention are body fluids such as blood, plasma, serum, amniotic fluid, lymph, urine, saliva, semen, gastric fluid, sputum, tears as well as tissue samples such as Chorinic Villus Samples (CVS) etc.

Further in the context of this invention, the terms "hybridisation" and "hybridisable" refer to hydrogen bonding, which may be Watson-Crick, Hoogsteen or reversed Hoogsteen hydrogen bonding, between complementary nucleoside or nucleotide bases. For example, adenine and thymine are complementary nucleobases, which pair through the formation of hydrogen bonds.

"Complementary," as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "hybridisable" and "complementary" are terms which are used to indicate a sufficient degree of complementarity or precise pairing such that stable and specific binding occurs between the oligonucleotide and the DNA or RNA target.

The expression "partially homologous" or "partially identical" refers to a relationship or degree of identity between two sequences, here preferably two marker sequences, each having a part of its sequence homologous to a part of the other. The homology between said parts is defined as at least about 90 or more preferable about 95% of the nucleotides match over said parts. Partially homologous therefore allows a low homology over the entire length of the sequences, as long as two defined parts exhibit a high homology of at least about 90 % or higher.

The expression "substantially homologous" or "substantially identical" refers to a relationship or degree of identity between two sequences, here preferably a primer and a marker sequence, and requires that at least about 85%, preferably at least about 90%, and most preferably at least about 95 or even more preferably about 100% of the nucleotides match over the defined length of the DNA sequences. Sequences that are substantially homologous can be identified by comparing the sequences using standard software available in sequence data banks, or in a Southern hybridisation experiment under, for example, stringent conditions as defined for that particular system. Defining appropriate hybridisation conditions is within the skill of the art and guidance can be found in literature, e.g. in
Non Patent Citation 0008: SAMBROOK, J. Molecular Cloning:A Laboratory Manual. 3, : Cold Spring Harbor Laboratory Press, 2001. ISBN 0879695773.

"Homology" or degree of identity can also be determined using applicable software, known and available to persons skilled in the art. Examples of such software include, but are not limited to ClustaIW (available for download at the website http://www.ebi.ac.uk/clustalw/) and NCBI BlastAlign (available at the website http:/www.bio.ic.ac.uk/resarch/belshaw/BlastAlign.tar).

The inventors here present a technique using sex chromosome-specific DNA sequences or marker sequences that are partially homologous to sequences specific for a second autosomal chromosome. These DNA sequences can be utilized for relative and absolute gene and/or chromosome quantification using methods and diagnostic kits as defined in the attached claims, hereby incorporated in their entirety. The utilization of the here described technique enables the quantification of genes and or chromosomes irrespectively of whether individuals are homozygous or carrying true chromosomal aneuploidies. This is a significant advantage compared to previous techniques which are associated with the risk of misdiagnosing homozygous individuals. Most important is the ability to quantify both genes and chromosomes using molecular biology techniques. This makes available completely novel possibilities to detect homozygous and heterozygous monogeneic and multigeneic disorders as well as chromosomal aneuploidies using relative quantitative and true quantitative molecular biology techniques.

The invention comprises the design or selection of at least two oligonucleotide sequences that are substantially homologous to genomic sequences present on at least two chromosomes of interest. The oligonucleotide sequences are further designed or chosen so that the genomic sequences between the oligonucleotide sequences on the chromosome of interest are partially homologous, thus resulting in amplification of fragments of separate sizes and/or separate nucleotide sequences. These amplified fragments may be directly quantified using true quantitative molecular techniques such as real-time PCR where the two genomic sequences are distinguished by their partially non-homologous nucleotide sequences. These amplified fragments can also be quantified using relative molecular quantification techniques as for instance QF-PCR where the amplified nucleotide fragments are distinguished by the separate sizes and/or nucleotide sequences of the amplified fragments as determined in a post-amplification detection step.

Both quantifying techniques, QF-PCR and real-time PCR, rely on comparable PCR amplification efficiency of all oligonucleotides included in the reaction. PCR, an abbreviation for Polymerase Chain Reaction, is a technique to exponentially amplify a small quantity of a specific nucleotide sequence in the presence of template sequence, two oligonucleotide primers that hybridize to opposite strands and flank the region of interest in the target DNA, and a thermostable DNA polymerase. The reaction is subjected to different temperatures in cycles involving template denaturation, primer annealing, and the extension of the annealed primers by DNA polymerase until enough copies are made for further analysis. The performing of PCR analyses per se is considered well known to a skilled person, having access to reagents, apparatuses and protocols from many different suppliers.

According to the invention, the marker sequences are partially homologous and of different length, the length difference being sufficient to distinguish the amplification products during detection.

According to the invention, the marker sequences are partially homologous but being sufficiently different in sequence to distinguish the amplification products by the sequence in between the PCR primers.

The method according to the invention is used for detection and/or diagnosis of the chromosomal monosomy Turner's syndrome (X0).

In an example used to demonstrate the utility of the invention, the pair of marker sequences were the BRAF-gene on chromosome 7 (BRAF7), and the BRAF2-gene on chromosome X (BRAFX). In that example, the marker sequences were amplified using the primers shown in the examples as SEQ ID NO. 1 and SEQ ID NO. 2 (See below).

Additional PCR primer sequences and suggested combinations for simultaneous amplification of BRAF7 and BRAFX include, but are not limited to the following:

Forward primer: GGGGAACGGAACTGATTTTT (SEQ ID NO 1)
Reverse primer: TGTTGGGCAGGAAGACTCTAA (SEQ ID NO 2)
Reverse primer: TTGTTGGGCAGGAAGACTCTA (SEQ ID NO 3)
Reverse primer: TGTTGGGCAGGAAGACTCTA (SEQ ID NO 4)
Reverse primer: GTGGTGACTTGGGGTTGCT (SEQ ID NO 5)
Forward primer: CTGGGGAACGGAACTGATT (SEQ ID NO 6)
Reverse primer: TGTTGGGCAGGAAGACTCTAA (SEQ ID NO 7)
Reverse primer: TTGTTGGGCAGGAAGACTCTA (SEQ ID NO 8)
Reverse primer TGGTGACTTGGGGTTGCT (SEQ ID NO 9)
Forward primer: CTGGGGAACGGAACTGATTT (SEQ ID NO 10)
Reverse primer: TTGTTGGGCAGGAAGACTC (SEQ ID NO 11)
Forward primer: TGGGGAACGGAACTGATTT (SEQ ID NO 12)
Forward primer: AACCCCAAGTCACCACAAAA (SEQ ID NO 13)
Reverse primer: TTGTGGTGACTTGGGGTTG (SEQ ID NO 14)
Reverse primer: TTTGTGGTGACTTGGGGTTG (SEQ ID NO 15)
Forward primer: CAACCCCAAGTCACCACAA (SEQ ID NO 16)
Reverse primer: GTGGTGACTTGGGGTTGC (SEQ ID NO 17)

The above sequences can be used in different combinations, for example as shown below:
1. SEQ ID NO 1 and SEQ ID NO 2.Expected product size (X-chromosome): 203 bp
2. SEQ ID NO 1 and SEQ ID NO 3.Expected product size (X-chromosome): 204 bp
3. SEQ ID NO 13 and SEQ ID NO 3.Expected product size (X-chromosome): 50 bp
4. SEQ ID NO 1 and SEQ ID NO 4.Expected product size (X-chromosome): 203 bp
5. SEQ ID NO 1 and SEQ ID NO 14.Expected product size (X-chromosome): 172 bp
6. SEQ ID NO 1 and SEQ ID NO 15.Expected product size (X-chromosome): 173 bp
7. SEQ ID NO 1 and SEQ ID NO 5.Expected product size (X-chromosome): 170 bp
8. SEQ ID NO 6 and SEQ ID NO 7.Expected product size (X-chromosome): 205 bp
9. SEQ ID NO 6 and SEQ ID NO 8.Expected product size (X-chromosome): 206 bp
10. SEQ ID NO 1 and SEQ ID NO 17.Expected product size (X-chromosome): 170 bp
11. SEQ ID NO 1 and SEQ ID NO 9.Expected product size (X-chromosome): 169 bp
12. SEQ ID NO 10 and SEQ ID NO 7.Expected product size (X-chromosome): 205 bp
13. SEQ ID NO 10 and SEQ ID NO 8.Expected product size (X-chromosome): 206 bp
14. SEQ ID NO 1 and SEQ ID NO 11.expected product size (X-chromosome): 204 bp
15. SEQ ID NO 12 and SEQ ID NO 7.Expected product size (X-chromosome): 204 bp
16. SEQ ID NO 12 and SEQ ID NO 8.Expected product size (X-chromosome): 205 bp
17. SEQ ID NO 13 and SEQ ID NO 11.Expected product size (X-chromosome): 50 bp
18. SEQ ID NO 6 and SEQ ID NO 14.Expected product size (X-chromosome): 174 bp
19. SEQ ID NO 16 and SEQ ID NO 7.Expected product size (X-chromosome): 50 bp
20. SEQ ID NO 16 and SEQ ID NO 3.Expected product size (X-chromosome): 51 bp

The primers can be distinguished not only by differences in length, but may also contain suitable marker functionalities, such as fluorescent markers or the like, well known to a person skilled in the art.

An embodiment of the present invention further makes available a diagnostic kit including reagents for performing the method defined above.

**Examples**

### 1. Design and amplification of nucleotide sequences used to distinguish sex chromosome aneuploidies using QF-PCR

In order to verify the ability of the invention to quantify the number of chromosomes in an unknown sample the following experimental conditions were used. PCR primers with sequences homologous to sequences present in the BRAF-gene on chromosome 7 (NCBI Acc. No NC_000007) as well as in the BRAF2-gene on chromosome X (NCBI Acc. No NC_000023) were designed. The PCR primer sequences used for amplification were as follows:
Forward primer: **5'-GGGGAACGGAACTGATTTTT-3'** (SEQ ID NO 1)
Reverse primer: **5'-HEX-TGTTGGGCAGGAAGACTCTAA-3'** (SEQ ID NO 2).

These PCR primer sequences were used to amplify a fragment of approximately 182 bp from chromosome 7 and a fragment of approximately 203 bp from chromosome X, respectively. PCR primers for the following genetic markers were always included in the multiplex PCR reaction: AMEL , DXS1187, SRY, DXS981 and XHPRT (see table I for details). A skilled person will be able to identify additional markers using routine experimentation *in silico.*

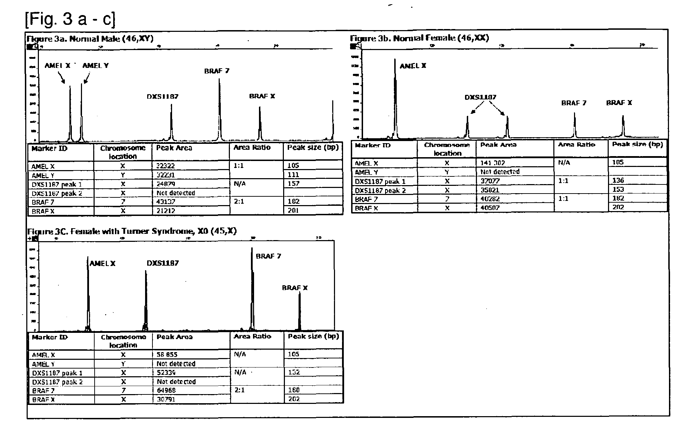

The DNA purification and PCR reactions were set up and performed as follows: Cells were obtained by amniocentesis or by cell culture. Cells were enriched and washed using standard centrifugation and PBS. Following enrichment and washing, DNA was extracted and purified using QIAamp DNA Blood Kit (Qiagen, Germany) and InstaGene Matrix (Bio-Rad Laboratories, UK). Purified nucleic acids were subsequently subjected to PCR amplification as described below. In brief, 5 µl of DNA (1-10 ng /µl) was added to the PCR reaction containing Taq-polymerase (2U/reaction), PCR primers (0,02 µM forward and reverse primers, respectively) and a buffer containing 50 mM KCl, 15 mM Tris-HCl pH 8.0. The sample was subjected to PCR amplification using Thermal Cycler GeneAmp^{®} PCR System 9700 using the following conditions; 95°C 15 min; 94°C 30 sec; 58°C 90 sec; 72°C 90 sec for 26 cycles, 72°C 30 min and 4°C forever. 3µl of the denatured and amplified sample was subsequently analysed on an ABI PRISM^{®} Genetic Analyzer as described in the addendum; ABI PRISM^{®} Genetic Analyzers User Manual. Gene-Scan-500 ROX was used as internal size standard. The results of such amplified and separated PCR fragments are shown in Figures 3a-c.
[Table 0001]
[Table]

**Table 1: Examples of genetic markers**

| **Marker ID** | **Location** | **Type** | **NCBI Acc. No** |
|---|---|---|---|
| BRAF | Chr X; Chr 7 | Non variable | X:NC_000007 |
| | | | Y:NC_000023 |
| AMEL | Chr X; Chr Y | Non variable | X:NC_000023 |
| | | | Y:NC_000024 |
| DXS1187 | Chr X | STR | NC_000023 |
| DXS981 | Chr X | STR | NC_000023 |
| SRY | Chr Y | Non variable | NC_000024 |
| XHPRT | Chr X | STR | NC_000023 |

A total of 303 clinical samples were analysed, whereof 94 blood samples, 204 amniotic fluid samples and 5 cell lines. The samples were analysed using the experimental conditions and the diagnostic kit described below. In addition, the amniotic fluid samples were also analyzed in parallel using karyotyping.

58 of the blood samples were determined to be male with all X-chromosomal STR markers homozygous and the BRAF (7:X) marker displaying an expected 2:1 ratio. 36 of the blood samples were determined female with at least one X-chromosomal STR markers heterozygous and with the expected 1:1 BRAF (7:X) ratio. One female sample was homozygous for all X-chromosomal STR markers tested but displayed a normal female 1:1 BRAF (7:X) ratio. Results from all tested blood samples are summarised in Tables 2a (female blood samples) and 2b (male blood samples).

A total of 102 amniotic fluid samples and cell lines were independently determined as females by QF-PCR and karyotyping (table 3a and figure 3b). Moreover, two of the female samples were independently determined as 45,X (table 3a and figure 3c) by QF -PCR and karyotyping. All X-chromosomal STR markers were homozygous and the BRAF (7:X) marker showed an abnormal female 2:1 ratio in QF-PCR for both 45,X samples. A total of 107 amniotic fluid samples and cell lines were independently determined as male by QF-PCR and karyotyping (Table 3b and Figure 3a). Moreover, two of the male samples were determined as 47,XXY by QF-PCR and karyotyping. At least one X-chromosomal STR marker was heterozygous and the BRAF (7:X) marker showed an abnormal male 1:1 ratio in QF-PCR for both 47,XXY samples.

**Results from QF-PCR in 94 blood samples**
[Table 0002]

**[Table]**

| **Table 2a. Female blood samples** | | |
|---|---|---|
| | **7 : X ratio** | |
| | **2:1** | **1:1** |
| **Total** | 0 | 36 |
| **All X markers Homozygous** | 0 | 1 |
| **At least one X marker heterozygous** | 0 | 35 |
| **Y chromosome detected** | 0 | 0 |

| **Table 2b. Male blood samples** | | |
|---|---|---|
| | **7 : X ratio** | |
| | **2:1** | **1:1** |
| **Total** | 58 | 0 |
| **All tested X markers Homozygous** | 58 | 0 |
| **At least one X marker heterozygous** | 0 | 0 |
| **Y chromosome detected** | 58 | 0 |

**Results from QF-PCR in 209 amniotic fluid and cell line samples**

[Table 0003]
[Table ]

**Table 3a. Female amniotic fluid and cell line samples**

| | **7 : X ratio** | |
|---|---|---|
| | **2:1** | **1:1** |
| **Total** | 2* | 100** |
| **All tested X markers Homozygous** | 2* | 0 |
| **At least one X marker heterozygous** | 0 | 100** |
| **Y chromosome detected** | 0 | 0 |

| | | |
|---|---|---|
| * Karyotyping results: 45,X **Karyotyping results: 46,XX | | |

[Table 0004]
[Table ]

**Table 3b. Male amniotic fluid and cell line samples**

| | **7 : X ratio** | |
|---|---|---|
| | **2:1** | **1:1** |
| **Total** | 105* | 2** |
| **All tested X markers Homozygous** | 105* | 0 |
| **At least one X marker heterozygous** | 0 | 2** |
| **Y chromosome detected** | 105* | 2** |

| | | |
|---|---|---|
| * Karyotyping results: 46,XY ** Karyotyping results: 47,XXY | | |

### 2. Diagnostic kit

A diagnostic kit (Devyser Complete™, Devyser AB, Stockholm, Sweden) was used for fetal diagnosis of Turner's syndrome in amniotic fluid obtained from pregnant women.

The diagnostic kit included the following reagents: a PCR reagent mix (Mix2), containing primer sets for detection of BRAF, AMEL, DXS1187, SRY and XHPRT in a buffered Mg 2+ solution, and a PCR activator mix (PCR activator), containing DNA polymerase in a buffered solution. In addition the kit included a second PCR reagent mix (Mix1) for analysis of STR markers present on chromosomes 13, 18 and 21.

The diagnostic kit was used according to the inventive method. Briefly, the DNA purification was set up and performed as follows: Amniotic fluid was obtained by amniocentesis. Amniocytes were enriched from the amniotic fluid and washed using standard centrifugation and PBS. Following enrichment and washing, DNA was extracted and purified using QIAamp DNA Blood Kit (Qiagen, Germany) or InstaGene Matrix (Bio-Rad Laboratories, UK).

The PCR reactions were set up and performed as follows. PCR reaction mixes were prepared by addition of 10 µL PCR Activator to each Mix1 and Mix2, respectively. 20 µL of each of the reaction master mixes were subsequently distributed to PCR vials and 5 µL purified nucleic acid was added to each of Mix 1 and Mix2. Positive (Normal male genomic DNA) and Non-template controls were included in each run.

The samples were subjected to PCR amplification using a Thermal Cycler using the following conditions; 95°C 15 min; 94°C 30 sec; 58°C 90 sec; 72°C 90 sec for 26 cycles, 72°C 30 min and 4°C until termination of the run. 1.5 µl of the amplified sample was mixed with 15 µl deionised formamide, containing a suitable size standard, and subsequently analysed on an ABI PRISM^{®} 3130 Genetic Analyzer as described in the instructions for use provided with the diagnostic kit.

### 3. Quantitative and qualitative molecular analysis of genetic status in DNA

Although the above mentioned method to distinguish chromosomal aneuploidies in this application has been demonstrated using QF-PCR, the detection of such gene and/or chromosomal aberration using nucleic acids can also be performed using other adequate molecular techniques such as: end-point PCR detection (including for example QF-PCR, PCR combined with detection using gel analysis, DNA arrays or MALDI-TOF etc), real-time detection PCR (including for example Dual-labelled probes, self-probing amplicons, intercalating dyes etc). These techniques are well known to persons skilled in the art, and the apparatuses, reagents and kits are commercially available from several suppliers. Given the teaching in the present description, examples and claims, a skilled person can adapt existing protocols and perform the invention.

### References

- HULTEN, M A, et al. Rapid and simple prenatal diagnosis of common chromosome disorders: advantages and disadvantages of the molecular methods FISH and QF-PCR. Reproduction. 2003, vol.126, no.3, p.279-97.
- NICOLINI, U, et al. The introduction of QF-PCR in prenatal diagnosis of fetal aneuploidies: time for reconsideration. Human Reproduction Update. 2004, vol.10, no.6, p.541-548.
- CAINE, A, et al. Prenatal detection of Down's syndrome by rapid aneuploidy testing for chromosomes 13, 18, and 21 by FISH or PCR without a full karyotype: a cytogenetic risk assessment. Lancet. 2005, vol.366, no.9480, p.123-8.
- NICOLINI, U, et al. The introduction of QF-PCR in prenatal diagnosis of fetal aneuploidies: time for reconsideration. Human Reproduction Update. 2004, vol.10, no.6, p.541-548.
- DONAGHUE, C, et al. Development and targeted application of a rapid QF-PCR test for sex chromosome imbalance. Prenat Diagn. 2003, vol.23, no.3, p.201-10.
- PENA, S D J. Fetal diagnosis of monosomy X (Turner's syndrome) with methylation-specific PCR. Prenatal Diagnosis. 2003, vol.23, p.769-770.
- CIRIGLIANO, V. X chromosome dosage by quantitative fluorescent PCR and rapid prenatal diagnosis of sex chromosome aneuploidies. Molecular Human reproduction. 2002, vol.8, no.11, p.1042-1045.
- SAMBROOK, J, et al. Molecular Cloning: A Laboratory Manual. 3rd edition. Cold Spring Harbor Laboratory Press, 2001. ISBN 0879695773.

## Claims

1. A method for quantification of chromosomes and genes in a sample taken from a human, in the diagnosis of Turners syndrome, **characterised in that**
a) at least two non-STR marker sequences are chosen, wherein
- one marker sequence is a sequence known to be present on the chromosome X and
- another marker sequence is a sequence known to be present on an autosomal chromosome,
b) the sample is amplified using at least two primers and wherein said at least two primers are complementary to the marker sequences known to be present on said chromosome X and said autosomal chromosome,
c) said marker sequences known to be present on said X-chromosome and said autosomal chromosome each having a part of its sequence homologous to a part of the other and the homology between said parts are at least 90% and wherein the at least two primers are specific for said part on the marker sequences and the marker sequences are of different length, the length difference being sufficient to distinguish the amplification products during detection,
d) amplified fragments are detected and a ratio of the amplification products of the marker sequences present on the autosomal chromosome and chromosome X which is 2:1 in a sample determined to be obtained from a female is indicative of Turners syndrome.

2. The method according to claim 1, wherein the at least two marker sequences known to be present on the X-chromosome and on an autosomal chromosome is the BRAF-gene on chromosome 7 and the BRAF2-gene on chromosome X.

3. The method according to claim 2, wherein the marker sequences are amplified using primers chosen among SEQ ID NO 1 - 17.

4. The method according to claim 3, wherein the marker sequences are amplified using a primer pair chosen among; SEQ ID NOs 1 and 2; SEQ ID NOs 1 and 3; SEQ ID NOs 1 and 4; SEQ ID NOs 1 and 5; SEQ ID NOs 1 and 9; SEQ ID NOs 1 and 11; SEQ ID NOs 1 and 14; SEQ ID NOs 1 and 15; SEQ ID NOs 1 and 17; SEQ ID NOs 6 and 7; SEQ ID NOs 6 and 8; SEQ ID NOs 6 and 14; SEQ ID NOs 10 and 7; SEQ ID NOs 12 and 7; SEQ ID NOs 13 and 3; SEQ ID NOs 13 and 11; SEQ ID NOs 16 and 3; and SEQ ID NOs 16 and 7.

5. The method according to claim 3, wherein the marker sequences are amplified using the primer pair SEQ ID NO. 1 and SEQ ID NO. 2.

6. The method according to claim 2, wherein the following further marker sequences are used: AMELX and AMELY, SRY, DXS1187, DXS981 and XHPRT.

7. A diagnostic kit including at least two primers chosen among SEQ ID NO 1 -17 for performing the method according to any one of claims 1 - 6.

## Patentansprüche

1. Verfahren zum Quantifizieren von Chromosomen und Genen in einer von einem Menschen genommenen Probe bei der Diagnose des Turner-Syndroms, **dadurch gekennzeichnet, dass**
a) wenigstens zwei Nicht-STR-Markersequenzen gewählt werden, wobei
- eine Markersequenz eine Sequenz ist, die bekanntermaßen auf dem X-Chromosom vorhanden ist, und
- eine andere Markersequenz eine Sequenz ist, die bekanntermaßen auf einem autosomalen Chromosom vorhanden ist;
b) die Probe amplifiziert wird, wobei man wenigstens zwei Primer verwendet und wobei die wenigstens zwei Primer komplementär zu den Markersequenzen sind, die bekanntermaßen auf dem X-Chromosom und auf dem autosomalen Chromosom vorhanden sind;
c) die Markersequenzen, die bekanntermaßen auf dem X-Chromosom und dem autosomalen Chromosom vorhanden sind, jeweils in Teilen ihrer Sequenz homolog zueinander sind und die Homologie zwischen den Teilen wenigstens 90% beträgt und wobei die wenigstens zwei Primer spezifisch für diesen Teil auf den Markersequenzen sind und die Markersequenzen von unterschiedlicher Länge sind, wobei der Längenunterschied ausreicht, um die Amplifikationsprodukte während des Nachweises voneinander zu unterscheiden;
d) amplifizierte Fragmente nachgewiesen werden und ein Verhältnis der Amplifikationsprodukte der Markersequenzen, die auf dem autosomalen Chromosom und dem X-Chromosom vorhanden sind, von 2:1 in einer Probe, die von einer weiblichen Probandin erhalten wurde, auf ein Turner-Syndrom hinweist.

2. Verfahren gemäß Anspruch 1, wobei es sich bei den wenigstens zwei Markersequenzen, die bekanntermaßen auf dem X-Chromosom und dem autosomalen Chromosom vorhanden sind, um das BRAF-Gen auf Chromosom 7 und das BRAF2-Gen auf dem X-Chromosom handelt.

3. Verfahren gemäß Anspruch 2, wobei die Markersequenzen unter Verwendung von Primern, die aus SEQ ID Nr. 1-17 ausgewählt sind, amplifiziert werden.

4. Verfahren gemäß Anspruch 3, wobei die Markersequenzen unter Verwendung eines Primerpaars amplifiziert werden, das aus folgenden ausgewählt ist: SEQ ID Nr. 1 und 2; SEQ ID Nr. 1 und 3; SEQ ID Nr. 1 und 4; SEQ ID Nr. 1 und 5; SEQ ID Nr. 1 und 9; SEQ ID Nr. 1 und 11; SEQ ID Nr. 1 und 14; SEQ ID Nr. 1 und 15; SEQ ID Nr. 1 und 17; SEQ ID Nr. 6 und 7; SEQ ID Nr. 6 und 8; SEQ ID Nr. 6 und 14; SEQ ID Nr. 10 und 7; SEQ ID Nr. 12 und 7; SEQ ID Nr. 13 und 3; SEQ ID Nr. 13 und 11; SEQ ID Nr. 16 und 3; und SEQ ID Nr. 16 und 7.

5. Verfahren gemäß Anspruch 3, wobei die Markersequenzen unter Verwendung des Primerpaars SEQ ID Nr. 1 und SEQ ID Nr. 2 amplifiziert werden.

6. Verfahren gemäß Anspruch 2, wobei die folgenden weiteren Markersequenzen verwendet werden: AMELX und AMELY, SRY, DXS1187, DXS981 und XHPRT.

7. Diagnostik-Kit, der wenigstens zwei Primer umfasst, die aus SEQ ID Nr. 1-17 ausgewählt sind, zur Durchführung des Verfahrens gemäß einem der Ansprüche 1 bis 6.

## Revendications

1. Procédé de quantification de chromosomes et de gènes dans un échantillon prélevé chez un sujet humain dans le diagnostic du syndrome de Turner, **caractérisé en ce que**
a) au moins deux séquences de marqueurs non STR sont choisies, dans lesquelles
- une séquence de marqueur est une séquence connue pour être présente sur le chromosome X et
- une autre séquence de marqueur est une séquence connue pour être présente sur un chromosome autosome,
b) l'échantillon est amplifié en utilisant au moins deux amorces, lesdites au moins deux amorces étant complémentaires des séquences de marqueurs connues pour être présentes sur ledit chromosome X et sur ledit chromosome autosome,
c) lesdites séquences de marqueurs connues pour être présentes sur ledit chromosome X et ledit chromosome autosome ayant chacune une partie de leur séquence qui est homologue à une partie de l'autre séquence et l'homologie entre lesdites parties est au moins égale à 90%, lesdites deux amorces au moins étant spécifiques à ladite partie des séquences de marqueurs et les séquences de marqueurs ayant des longueurs différentes, la différence de longueur étant suffisante pour distinguer les produits d'amplification lors de la détection,
d) les fragments amplifiés sont détectés et un ratio des produits d'amplification des séquences de marqueurs présentes sur le chromosome autosome et sur le chromosome X égal à 2:1 dans un échantillon dont il est déterminé qu'il provient d'une femme est un indicateur du syndrome de Turner.

2. Procédé selon la revendication 1, dans lequel lesdites au moins deux séquences de marqueurs connues pour être présentes sur le chromosome X et sur un chromosome autosome est le gène BRAF sur le chromosome 7 et le gène BRAF2 sur le chromosome X.

3. Procédé selon la revendication 2, dans lequel les séquences de marqueurs sont amplifiées en utilisant des amorces choisies parmi les SEQ ID N° 1 à 17.

4. Procédé selon la revendication 3, dans lequel les séquences de marqueurs sont amplifiées en utilisant une paire d'amorces choisie parmi les paires suivantes : SEQ ID N° 1 et 2 ; SEQ ID N° 1 et 3 ; SEQ ID N° 1 et 4 ; SEQ ID N°1 et 5 ; SEQ ID N° 1 et 9 SEQ ID N° 1 et 11 ; SEQ ID N° 1 et 14 ; SEQ ID N° 1 et 15 ; SEQ ID N° 1 et 17 ; SEQ ID N°6 et 7 ; SEQ ID N°6 et 8 ; SEQ ID N° 6 et 14 ; SEQ ID N°10 et 7 ; SEQ ID N°12 et 7 ; SEQ ID N°13 et 3 ; SEQ ID N° 13 et 11 ; SEQ ID N° 16 et 3 ; et SEQ ID N°16 et 7.

5. Procédé selon la revendication 3, dans lequel les séquences de marqueurs sont amplifiées en utilisant la paire d'amorces SEQ ID N° 1 et SEQ ID N° 2.

6. Procédé selon la revendication 2, dans lequel les autres séquences de marqueurs suivantes sont utilisées : AMELX et AMELY, SRY, DXS1187, DXS981 et XHPRT.

7. Kit de diagnostic comprenant au moins deux amorces choisies parmi les SEQ ID N° 1 à 17 pour mettre en oeuvre le procédé selon l'une quelconque des revendications 1 à 6.
